(19) Europäisches Patentamt
European Patent Office
Office européen des brevets

(11) Publication number: **0 486 809 A2**

(12) **EUROPEAN PATENT APPLICATION**

(21) Application number: **91117526.3**

(51) Int. Cl.$^5$: **A61K 31/185**

(22) Date of filing: **15.10.91**

(30) Priority: **14.11.90 GB 9024738**

(43) Date of publication of application: **27.05.92 Bulletin 92/22**

(84) Designated Contracting States: **DE GB IT**

(71) Applicant: **FARMITALIA CARLO ERBA S.r.L.**
**Via Carlo Imbonati 24**
**I-20159 Milano(IT)**

(72) Inventor: **Alzani, Rachele**
**Via Eustachi 30**
**I-20129 Milan(IT)**
Inventor: **Ciomei, Marina**
**Voa Del Molo 1**
**I-27020 Torre d'Isola (Pavia)(IT)**
Inventor: **Grazioli, Laura**
**Via Gavinana 8**
**I-21052 Busto Arsizio (Varese)(IT)**
Inventor: **Marcucci, Fabrizio**
**Piazza del Carroccio 23**
**I-20025 Legnano (Milan)(IT)**

(74) Representative: **Ferrario, Vittorino**
**c/o Farmitalia Carlo Erba S.r.l. Via Carlo Imbonati 24**
**I-20159 Milano(IT)**

(54) **Use of suramin against TNF-related diseases.**

(57) Suramin or a pharmaceutically acceptable salt thereof is useful as tumor necrosis factor $\alpha$-inhibitor. Suramin may therefore be used in the prophylaxis and/or treatment of diseases in which tumor necrosis factor $\alpha$ plays a detrimental role, such as cachexia, septic shock, AIDS, rheumatoid arthritis, graftversus-host disease, cerebral malaria, myocardial ischemia and reperfusion, bactericidal granulomas, adult respiratory distress syndrome and silica-induced pulmonary fibrosis, acute and chronic inflammatory diseases.

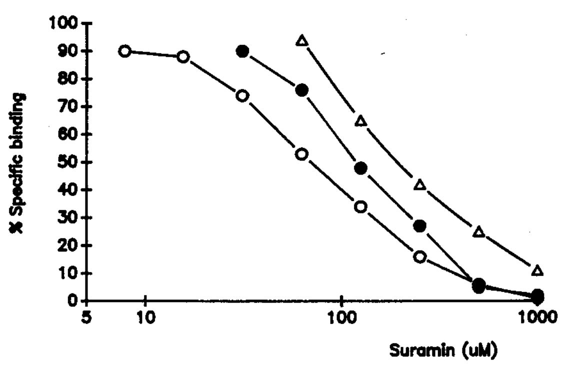


Fig. 1

EP 0 486 809 A2

The present invention concerns a new therapeutic application of suramin.

Tumor Necrosis Factor a (TNFα) is a polypeptide of molecular weight 17500, whose primary and tertiary structure has been elucidated (Pennica, D. et al. Nature 312 : 724 (1984) and Jones, E.Y. et al. Nature 338 : 225 (1989)).

TNFα is biologically active as a dimer or trimer. The main source of TNFα are cells of the monocyte/macrophage lineage, but after appropriate stimulation it is produced by other cell types as well (e.g. T-lymphocytes).

TNFα was originally described as a molecule that induces haemorragic necrosis of tumors in mice. However, after large amounts of homogeneous TNFα became available upon cloning of its cDNA, it soon became clear that TNFα mediates a large array of biological activities that allow the definition of TNFα as a mediator of inflammation. See, generally, Beutler, B. and Cerami, A., Nature 320 : 584 (1986). Accordingly, TNFα plays, within the inflammatory response, a pivotal role in the host defense against invasion of the organism by noxious agents. However, under certain circumstances, like chronic or acute, systemic or localized hyperproduction, TNFα leads, in conjunction with other mediators of the inflammatory response, to a large number of pathologic conditions. Cachexia and septic shock are the best known examples (see, Beutler, B. and Cerami, A., Supra and Oliff, A., et al., Cell 50 : 555 (1987). Likewise, TNFα is supposed to play a pathogenic role in AIDS (Folks, T.M., et al., Proc. Natl. Acad. Sci. USA 86 : 2365 (1989)), in graft-versus-host disease (Piguet, P.F., et al. J. Exp. Med. 166 : 1280 (1987)), in cerebral malaria (Grau, G.E., et al. Science 237 : 1210 (1987)), rheumatoid arthritis (Brennan, F.M., et al., Lancet ii : 244 (1989)) myocardial ischemia and reperfusion (Lefer, A.M., et al. Science 249 : 61 (1990)), development of bactericidal granulomas (Kindler, V. et al. Cell 56 : 731 (1989)), adult respiratory distress syndrome (Millar, A.B. et al. Lancet ii : 712 (1989)), silica - induced pulmonary fibrosis (Piguet, P.F. et al. Nature 344 : 245 (1990)-), tumor metastasisation (Malik, S.T.A. et al. Eur. J. Cancer 26 : 1031 (1990)), hypercoagulable states (Bauer, K.A. et al. Blood 74 : 165 (1989)) and renal diseases (Bertani, T. et al. Am. J. Path. 134 : 419 (1989)). This has suggested that agents that inhibit TNFα activity (TNFα inhibitors) can be therapeutically useful in those disease states in which TNFα has been shown to play a detrimental role.

Several TNFα inhibitors have been described in the last few years : monoclonal antibodies (Tracey, K.J. et al. Nature 330 : 662 (1987)), tryptophan and indole (Yuhas, Y. et al. Eur. Cytokine Net. 1 : 35 (1990)), α-melanocyte-stimulating hormone (Mason, M.J. and Van Epps, D. J. Immunol. 142 : 1646 (1989)), epidermal growth factor and transforming growth factor (Sugarman, B.J. et al. Cancer Res. 47 : 780 (1987)) and uromodulin (Sherblom, A.P. et al. J. Biol. Chem. 263 : 5418 (1988)).

Now we have found that suramin acts as a potent TNFα-inibitor and accordingly it can be used as a prophylactic and/or therapeutic agent in those disease states in which TNFα exerts a pathogenic effect. Accordingly, the present invention provides the use of suramin, or a pharmaceutically acceptable salt thereof, in the preparation of a medicament for use in the prophylaxis and/or treatment of a disease state in which Tumor Necrosis Factor α exerts a pathogenic effect.

Suramin is 8,8' - (carbonylbis(imino-3,1-phenylenecarbonylimino (4-methyl-3,1-phenylene) carbonylimino)) bis-1,3 ,5-naphthalenetrisulfonic acid (GB Patent No. 224849). This polyanionic compound has been used for many decades as a prophylactic and therapeutic agent for trypanosomiasis. It was subsequently shown that suramin is able to block the activity of a variety of proteins like cellular and viral enzymes and growth factors (Mitsuya, M. et al. Science 226 : 172 (1984), Hosang, M. J. Cell. Biochem. 29 : 265 (1985), De Clercq, E. Cancer Lett. 8 : 9 (1979)). Given these activities it is currently under evaluation as an antitumor agent and clinical trials are being performed.

Examples of pharmaceutically acceptable salts of suramin are either those with inorganic bases, such as sodium, potassium, calcium and aluminium hydroxides, or with organic bases, such as lysine, triethylamine, triethanolamine, dibenzylamine, methylbenzylamine, di-(2-ethylhexyl)-amine, piperidine, N-ethylpiperidine, N,N-diethylaminoethylamine, N-ethylmorpholine, β-phenethylamine, N-benzyl-β-phenethylamine, N-benzyl-N, N-dimethylamine and the other acceptable organic amines.

We have employed 2 different assays in order to demonstrate that suramin acts as a TNF α-inhibitor. First, we have studied the capacity of suramin to inhibit the binding of human TNFα to specific cell-surface receptors. Second, we have studied the capacity of suramin to inhibit the cytotoxic activity of human TNF α on a cell line in vitro.

In the accompanying figures

Figure 1 shows the inhibition, by suramin, of the binding of different concentrations of human TNFα to human K562 cells. Inhibitory concentrations $_{50}$ ($IC_{50}$) of suramin were 78 μm, 126 μM and 180 μm for 2.5 x $10^{-11}$M

(o—o),

$10^{-10}$M

(●—●)

and 4 x $10^{-10}$M (△-△) TNFα, respectively.

Figure 2 shows the inhibition, by suramin, of the binding of a concentration of 3 x $10^{-10}$M human TNFα to mouse LM cells. The $IC_{50}$ was 175 µM.

Figure 3 shows the inhibition, by suramin, of the binding of $10^{-10}$M human TNFα to K562 cells.

In this experiment suramin and TNFα were added simultaneously to the cells (o-o). Alternatively suramin and TNFα were mixed and incubated for 4 (●-●) or 24 hours (△-△) before being added to the cells.

Figure 4 shows the effect of suramin on the binding of $10^{-10}$M human TNFα to K562 cells.

In this experiment suramin and TNFα were added either simultaneously to the cells (o-o) or sequentially (●-●).

Figure 5 shows the effect of different doses of suramin on the cytotoxic activity of different doses of human TNFα on mouse LM cells treated with actinomycin D.

Figure 6 shows the effect of different doses of suramin on the cytotoxic activity of different doses of human TNFα on untreated mouse LM cells.

In the experiment depicted in Figure 1 suramin was mixed, at the indicated doses, with 2.5 x $10^{-11}$M, $10^{-10}$M, or 4 x $10^{-10}$M iodinated human TNFα - (New England Nuclear, NEN, Wilmington, DE). The mixtures were then added immediately to K562 cells. The residual binding of TNF α was measured and compared to the binding to K562 cells in the absence or presence of a 100-fold higher dose of "cold" unlabeled human TNFα (Esquire Chemie AG, Zuerich, Switzerland). As can be seen from Figure 1 suramin is able to inhibit the binding of iodinated TNFα to human K562 cells. Higher doses of suramin are required to inhibit the binding of higher doses of iodinated TNFα.

In the experiment depicted in Figure 2 suramin was mixed, at the indicated doses, with $10^{-10}$M iodinated human TNFα. The mixtures were then added to mouse LM cells. The residual binding of TNFα was measured and compared to the binding to LM cells in the absence or presence of a 100-fold higher dose of "cold" unlabeled human TNFα. As can be seen from Figure 2 suramin is able to inhibit the binding of 3 x $10^{-10}$M iodinated TNF α to mouse LM cells as well.

In the experiment depicted in Figure 3 suramin was mixed at the indicated doses, with $10^{-10}$M iodinated human TNFα. The mixtures were either incubated for 4 or 24 hours or TNFα and suramin were added simultaneously to K562 cells. The residual binding of TNFα to K562 was measured and compared to the binding in the absence or presence of a 100-fold higher dose of "cold" unlabeled human TNFα.

As can be seen from Figure 3, preincubating suramin and TNFα for 24 hours significantly decreases the $IC_{50}$ of suramin.

In the experiment depicted in Figure 4 suramin and $10^{-10}$M iodinated human TNFα were added either simultaneously or sequentially to K562. When added sequentially, K562 cells were washed between the incubation with suramin and that with TNFα.

As can be seen from Figure 4, no inhibition of the binding of TNFα to K562 is observed in the sequential addition of suramin and TNFα.

Taken together, the results depicted in Figures 3 and 4 show that suramin exerts its inhibitory effect on the binding of TNFα to K562 cells through a direct action on TNFα and not through an action on TNFα-receptors on K562 cells.

In the experiment depicted in Figure 5 different concentrations of human TNFα (Esquire) were incubated, in the absence or presence of different doses of suramin, with mouse LM cells treated with actinomycin D. Treatment with actinomycin D is known to render LM cells more sensitive to the cytotoxic activity of TNFα (Ruff, M.R. and Gifford, G.E., J. Immunol. 125: 1671 (1980)). The cytotoxic activity of human TNFα was measured the day after.

The experiments depicted in Figure 6 were similar to the experiments of Figure 5, except that actinomycin D was omitted from the incubation mixture. In this case the cytotoxic activity of human TNFα was measured after 2 days. As can be seen, in both experiments suramin inhibited, in a dose-dependent manner the cytotoxic activity of human TNFα. The degree of inhibition was dependent on the activity of human TNFα employed, i.e. doses of human TNFα exerting high cytotoxic activity were less inhibitable than those exerting lower cytotoxic activity.

By virtue of the disclosed TNFα-inhibitory activity suramin or a pharmaceutically acceptable salt thereof can be employed in mammals, including humans, for prophylactic and/or therapeutic use in any disease state in which TNFα plays a detrimental role. Typically, such disease states are septic shock, graft-versus-host disease, cerebral malaria, cachexia, AIDS, rheumatoid arthritis, myocardial ischemia and reperfusion, bactericidal granulomas, adult respiratory distress syndrome, silica - induced pulmonary fibrosis, acute and chronic inflammatory disease states.

An effective amount of suramin or a pharmaceutically acceptable salt thereof is administered to a patient subject to or suffering from a disease state in which TNFα exerts a pathogenic

effect. Single or multiple administrations can be carried out with dose levels and patterns being selected by the treating physician. In any event, the pharmaceutical formulations should provide a quantity of suramin sufficient to treat effectively a said disease state or to prevent the onset of a said disease state. Doses of suramin ranging e.g. from about 20 to about 200 mg/kg of body weight can be used for parenteral administration in adult humans.

The present invention also provides products containing suramin, or a pharmaceutically acceptable salt thereof, and a different active agent, as a combined preparation for simultaneous, separate or sequential use in the treatment of a disease state in which TNFα exerts a pathogenic effect. A method of preventing or treating a disease state in which TNFα exerts a pathogenic effect may therefore comprise the combined administration of an effective amount of (1) suramin or a pharmaceutically acceptable salt thereof, and (2) a different active agent to a patient subject to or suffering from the said disease state.

The term "combined" method of treatment is meant to include both separate and substantially contemporaneous administration of suramin or a pharmaceutically acceptable salt thereof and a different pharmaceutically active agent. Therapeutically effective amounts of suramin or a salt thereof and a different active agent are administered, typically as pharmaceutical compositions.

Active agents that can be administered with suramin depend on the disease state. They include, for instance, gamma globulin, immunoglobulin and monoclonal antibody products, antibiotics and antimicrobial products. Typically, the antimicrobial agents may include a penicillin in conjunction with an aminoglycoside (e.g. gentamycin, tobramycin). However several well known additional agents, e.g. cephalosporins, can be utilized.

Suramin or a salt thereof and pharmaceutical compositions comprising suramin or a suramin salt can be administered orally or parenterally by subcutaneous, intramuscular, intraarterial or intravenous administration.

Pharmaceutical compositions containing suramin, either as the sole active agent or together with a different active agent, are usually prepared following conventional methods. The compositions, generally speaking, comprise carriers and/or diluents which include alcoholic/aqueous solutions, emulsions or suspensions, including saline or buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers, such as those based on Ringer's dextrose and the like. Preservatives and other additives can also be present such as, for example, antimicrobials, antioxidants, chelating agents, inert gases, and the like. See, generally, <u>Remington's Pharmaceutical Sciences</u> 16$^{th}$ Ed., Mack, eds., 1980.

In the following we describe the assays that have been employed in order to detect the TNFα inhibitory activity of suramin. It will be immediately appreciated by those of ordinary skill in the art that any other in vitro or in vivo TNFα-assay can employed in order to detect the TNFα -inhibitory activity of suramin. Therefore the following description of the assays is not intended to limit the invention in any manner.

1) <u>Inhibition by suramin of the binding of iodinated human TNF α to K562 cells.</u>

Iodinated human TNFα (NEN, specific activity ≈ 30 - 70 µCi/µg protein) is diluted so to have concentrations of 1.6 x $10^{-9}$M, 4 x $10^{-10}$M and $10^{-10}$M in RPMI 1640 medium (Flow, Opera, Italy) supplemented with 2% foetal calf serum (FCS, PBI, Milan, Italy) and 14 mM Hepes (Flow). This medium will hereon be referred to as binding medium (BM). "Cold" unlabeled human TNF α (Esquire) is diluted in BM so to have concentrations of 1.6 x $10^{-7}$M, 4 x $10^{-8}$M and $10^{-8}$M. Suramin (Bayer, Leverkusen, FRG) is dissolved in BM so to have a concentration of 4 mM. Starting from this solution a series of dilutions are set up in BM.

K562 cells (human chronic myelogenous leukemia, ATCC CCL243) are adjusted to a concentration of 2 x $10^{6}$/ml in BM. 100 µl of the cell suspension are then incubated with 50 µl of each iodinated TNFα concentration and with 50 µl BM or 50 µl of a 100 fold higher concentration of "cold" TNF α or 50 µl of each suramin dilution. The mixtures are incubated for 4 hours at 4°C. Thereafter the cells are centrifuged through a phthalate-dibutyl phthalate oil mixture (1:1,5 vol/vol, Fluka AG, Buchs, Switzerland). Cell-associated radioactivity is determined in a gamma-counter. Data thus obtained are processed by means of an equilibrium binding data analysis program (EBDA, Elsevir Science Publishers, Amsterdam, Netherlands).

In one series of experiments suramin and iodinated human TNFα are coincubated for 4 or 24 hours and then added to the cells. The experimental design is similar to the one described previously except that

- 100 µl of 2 x $10^{-10}$M iodinated human TNFα are mixed with 100 µl BM or 100 µl of a 100 fold higher concentration of "cold" TNFα or 100 µl of different suramin diluitions. The suramin diluitions are added either immediately or after 20 or 24 hours. The mixtures

are allowed to stand during the preincubation periods at 4° C. Thereafter the mixtures are added to a pellet of 2 x $10^5$ K562 cells and further incubated for 4 hours at 4° C.

In another series of experiments the effect of suramin on the TNFα-binding capacity of K562 cells is evaluated. The experimental design is similar to the first except that

- 100 µl of the K562 cell suspension are coincubated with 100 µl BM or with 100 µl of different suramin dilutions for 4 hours at 4° C. Thereafter the cells are centrifuged. Cells that had been preincubated with BM are resuspended in 200 µl $10^{-10}$M iodinated human TNFα with or without a 100 fold higher concentration of "cold" TNF. Cells that had been preincubated with the different suramin dilutions are resuspended in 200 µl $10^{-10}$M iodinated human TNFα alone. In parallel other K562 cells that had not been preincubated are resuspended in iodinated human TNFα with or without "cold" TNFα. Thereafter the mixtures are incubated for other 4 hours at 4° C and treated as described previously.

2) Inhibition by suramin of the binding of iodinated human TNF α to LM cells.

Iodinated human TNFα is diluted in BM so to have a concentration of 1.2 x $10^{-9}$M. "Cold" unlabeled human TNFα is diluted in BM so to have a concentration of 1.2 x $10^{-7}$M. Suramin is dissolved and diluted in BM as for the binding assay on K562 cells.

For the binding assay, 24-well plates (Costar, Vismara Associate, Trezzano sul Naviglio, Italy) are used, that have been seeded the day before with mouse LM cells (Rubin, B.Y. et al. J. Exp. Med. 162: 1099 (1985)), 200.000 cells/well. The medium is discarded and replaced with 100 µl BM supplemented with 0.04% Na $N_3$, 50 µl iodinated human TNF α and 50 µl BM or 50 µl cold TNF α or 50 µl of each suramin dilution. After 4 hours at 4° C cells are washed 4 x with BM, solubilized with 2% SDS and cell-associated radioactivity is determined. Data are processed as described before.

3) Inhibition by suramin of the cytotoxic activity of TNF α on untreated mouse LM cells.

On day O logarithmically growing mouse LM cells (Rubin, B.Y. et al. J. Exp. Med. 162 : 1099 (1985)) are trypsinized, centrifuged and resuspended at a final concentration of 5 x $10^4$ cells/ml of Eagle's minimun essential medium supplemented with 5% FCS and 1% glutamine (complete MEM). 100 µl of this suspension are added to each well of flat bottom microtiter plates (Falcon 3072, BD, Bridgwaterlane, NJ). The plates are then incubated overnight at 37° C, 5% $CO_2$. At this point a series of 2-fold dilutions of human TNFα are set up in complete MEM. Suramin is dissolved in complete medium so to have a concentration of 1 mM. Starting from this solution a series of dilutions are set up in complete MEM.

At this point 50 µl of each TNFα dilution and 50 µl of each suramin dilution are added to the wells.

To some wells 50 µl of each TNFα dilution and 50 µl of complete MEM are added. To some other wells 50 µl of complete MEM and 50 µl of each suramin dilution are added. Finally to some other wells only 100 µl of complete MEM are added. Thereafter the plates are incubated for 48 hours at 37° C.

At the end of this incubation period 20 µl of thiazolyl blue solution (MTT, Calbiochem, 50 mg/ml in PBS, La Jolla, California) are added to each well. The plates are further incubated for 4 hours at 37° C. Then, supernatants are discarded, 200 µl of dimethylsulfoxide (DMSO, Farmitalia Carlo Erba) are added to each well and the extent of color development is read on a plate ELISA reader at 570 nm. OD values obtained for each TNF α concentration and each TNFα-suramin mixture are expressed as percent (%) of those obtained for the controll wells containing only complete MEM and complete MEM plus each suramin dilution, respectively. Given the linear relationship between OD values and cell viability the % values are reported in Figure 2 as % viability.

Inhibition by suramin of the cytotoxic activity of TNF α on actinomycin D - treated LM cells.

The experimental design is similar to that for the assay on untreated LM cells.

The differences are as follows.

1) 100 µl of a cell-suspension containing 3 x $10^5$ cells/ml are added to each well of microtiter plates.

2) Before setting up the TNFα-cytotoxicity assay, the cells are pretreated for 4 hours at 37° C with complete MEM (100 µl/well) containing 2 µg/ml actinomycin D (Fluka).

3) The cytotoxic activity of human TNF α is measured after an incubation period of 24 hours at 37° C.

## Claims

1. The use of suramin, or a pharmaceutically acceptable salt thereof, in the preparation of a medicament for use in the prophylaxis and/or treatment of a disease in which Tumor Necrosis Factor α plays a detrimental role.

**2.** The use of suramin, according to claim 1, wherein the disease is chosen from the group including cachexia, septic shock, AIDS, rheumatoid arthritis, graft-versus-host disease, cerebral malaria, myocardial ischemia and reperfusion, bactericidal granulomas, adult respiratory distress syndrome and silica - induced pulmonary fibrosis, acute and chronic inflammatory diseases.

**3.** Products containing suramin, or a pharmaceutically acceptable salt thereof, and a different active agent, as a combined preparation for simultaneous, separate or sequential use in the treatment of a disease in which TNFα exerts a pathogenic effect.

**4.** Products, according to claim 3, wherein the different active agent is chosen from gamma globulins, immunoglobulins, monoclonal antibodies, antibiotics and antimicrobial products.

**5.** Products according to claim 3 or 4, wherein the disease state is selected from cachexia, septic shock, AIDS, rheumatoid arthritis, graft-versus-host disease, cerebral malaria, myocardial ischemia and reperfusion, bactericidal granulomas, adult respiratory distress syndrome and silica-induced pulmonary fibrosis, acute and chronic inflammatory diseases.

**6.** An agent for use in the prophylaxis and/or treatment of a disease in which Tumor Necrosis Factor α plays a detrimental role comprising suramin or a pharmaceutically acceptable salt thereof.

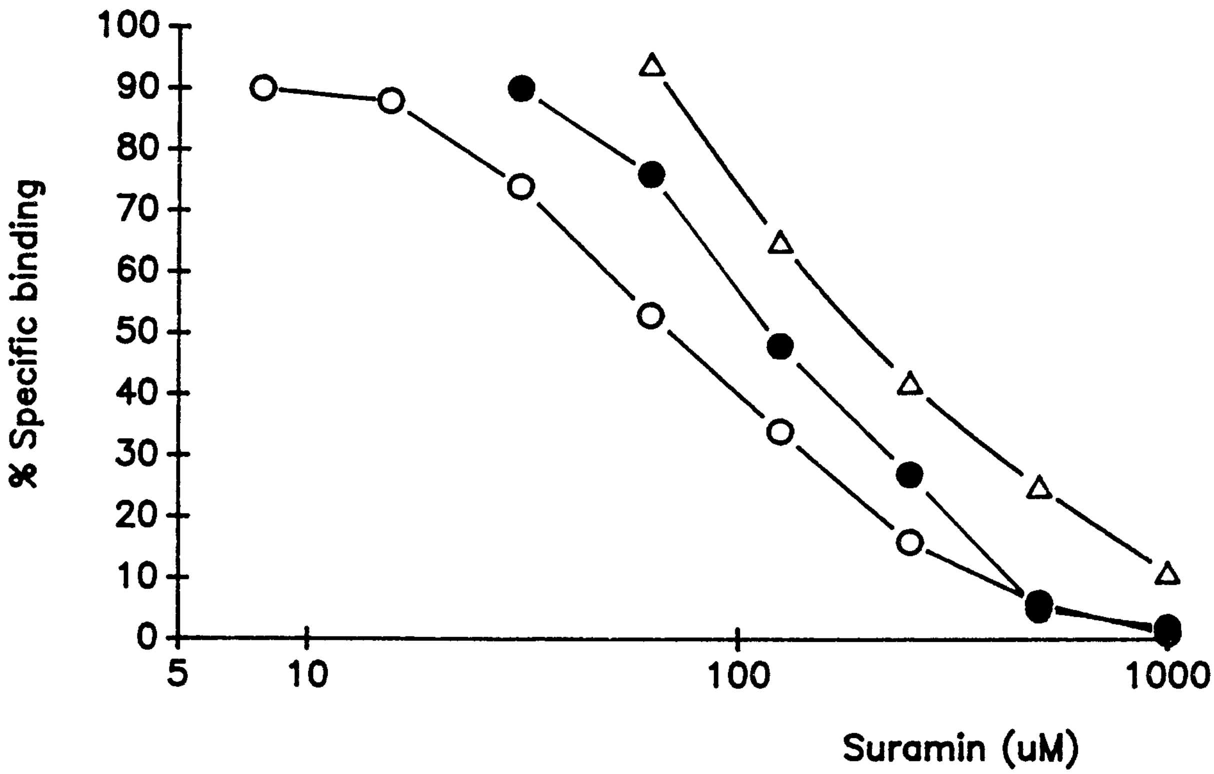
100
90
80
70
60
50
40
30
20
10
0
% Specific binding
5
10
100
1000
Suramin (uM)

Fig. 1

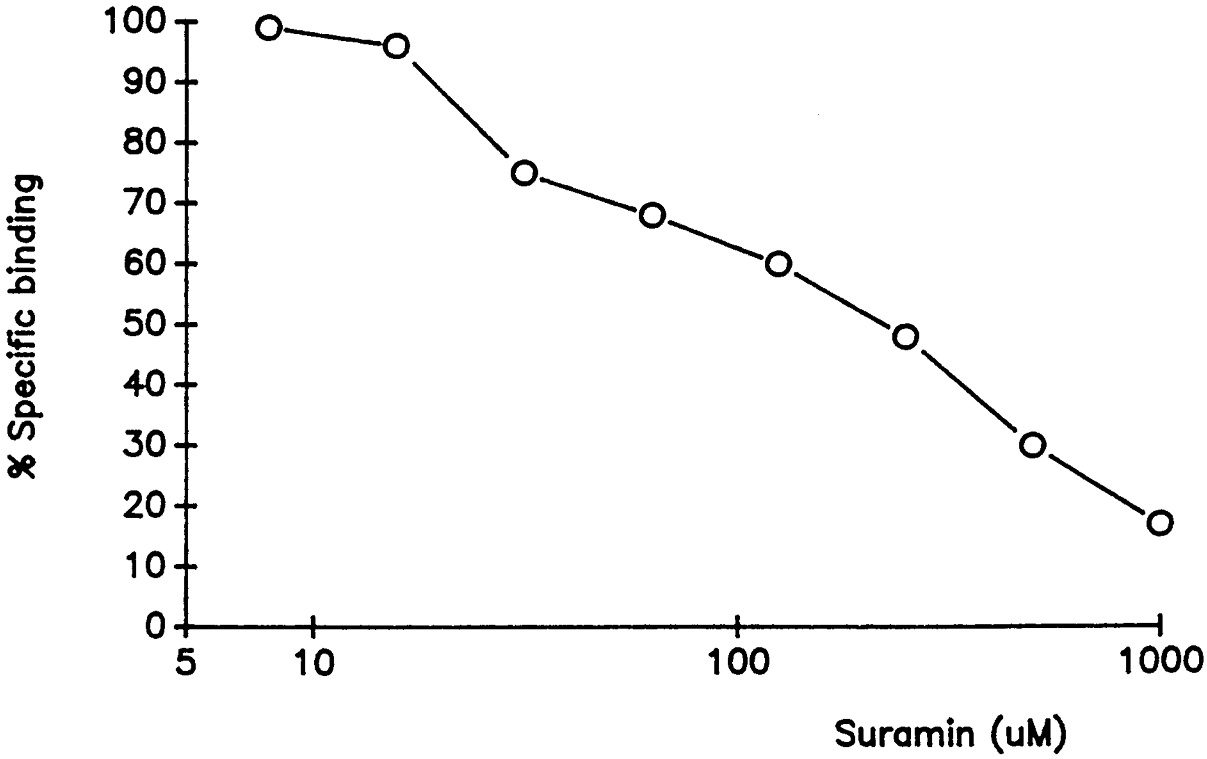
100
90
80
70
60
50
40
30
20
10
0
% Specific binding
5
10
100
1000
Suramin (uM)

Fig. 2

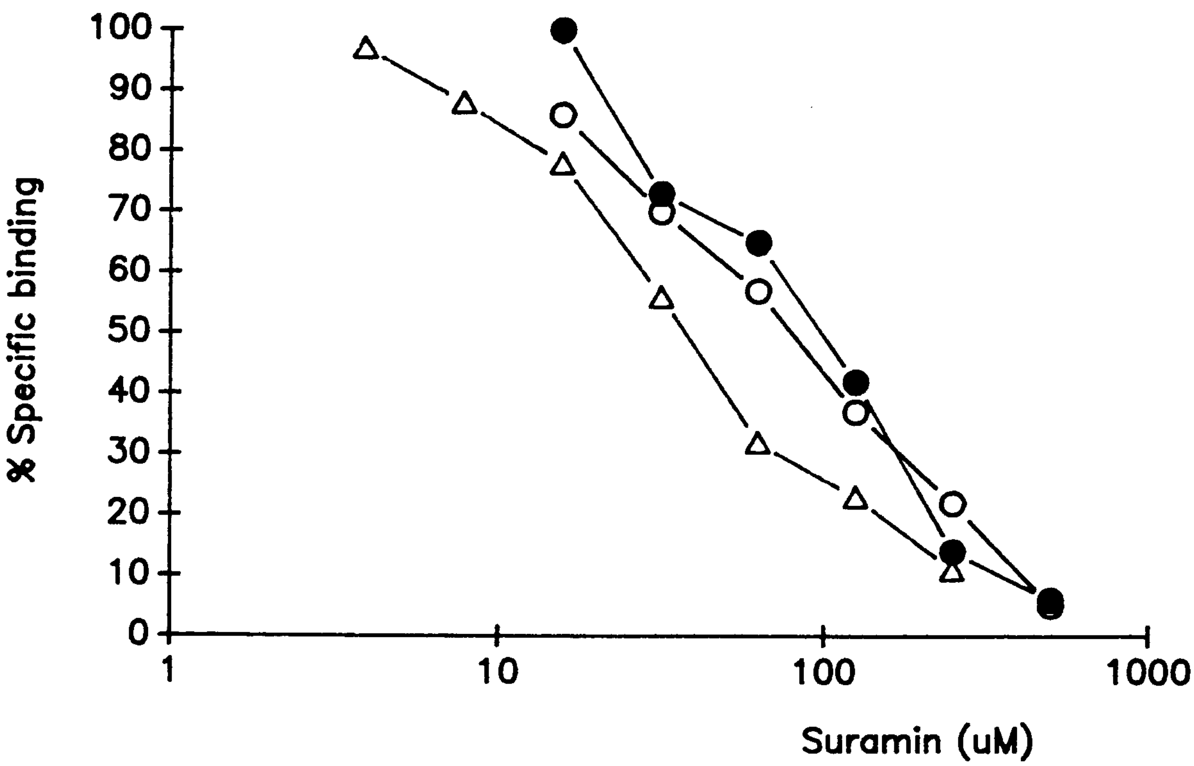
100
90
80
70
60
50
40
30
20
10
0
% Specific binding
1
10
100
1000
Suramin (uM)

Fig. 3

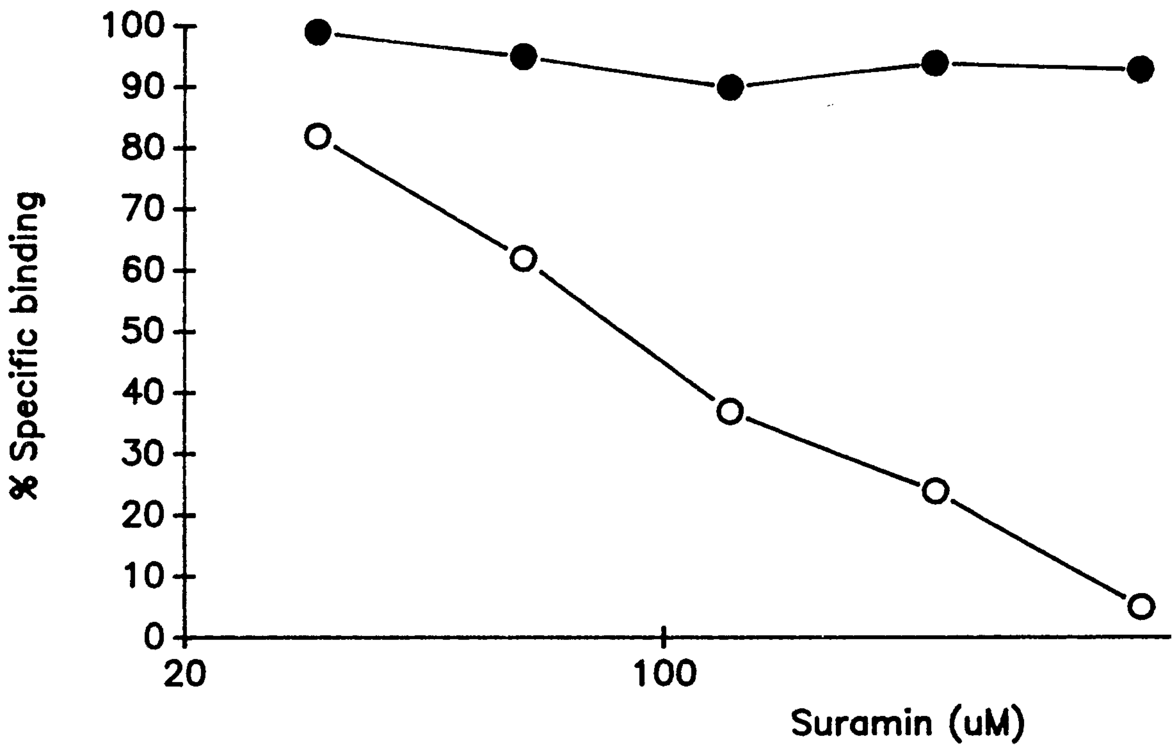

100
90
80
70
60
50
40
30
20
10
0
% Specific binding
20
100
Suramin (uM)


Fig. 4

Effect of Suramin on TNF cytotoxicity on LM cells + ActD

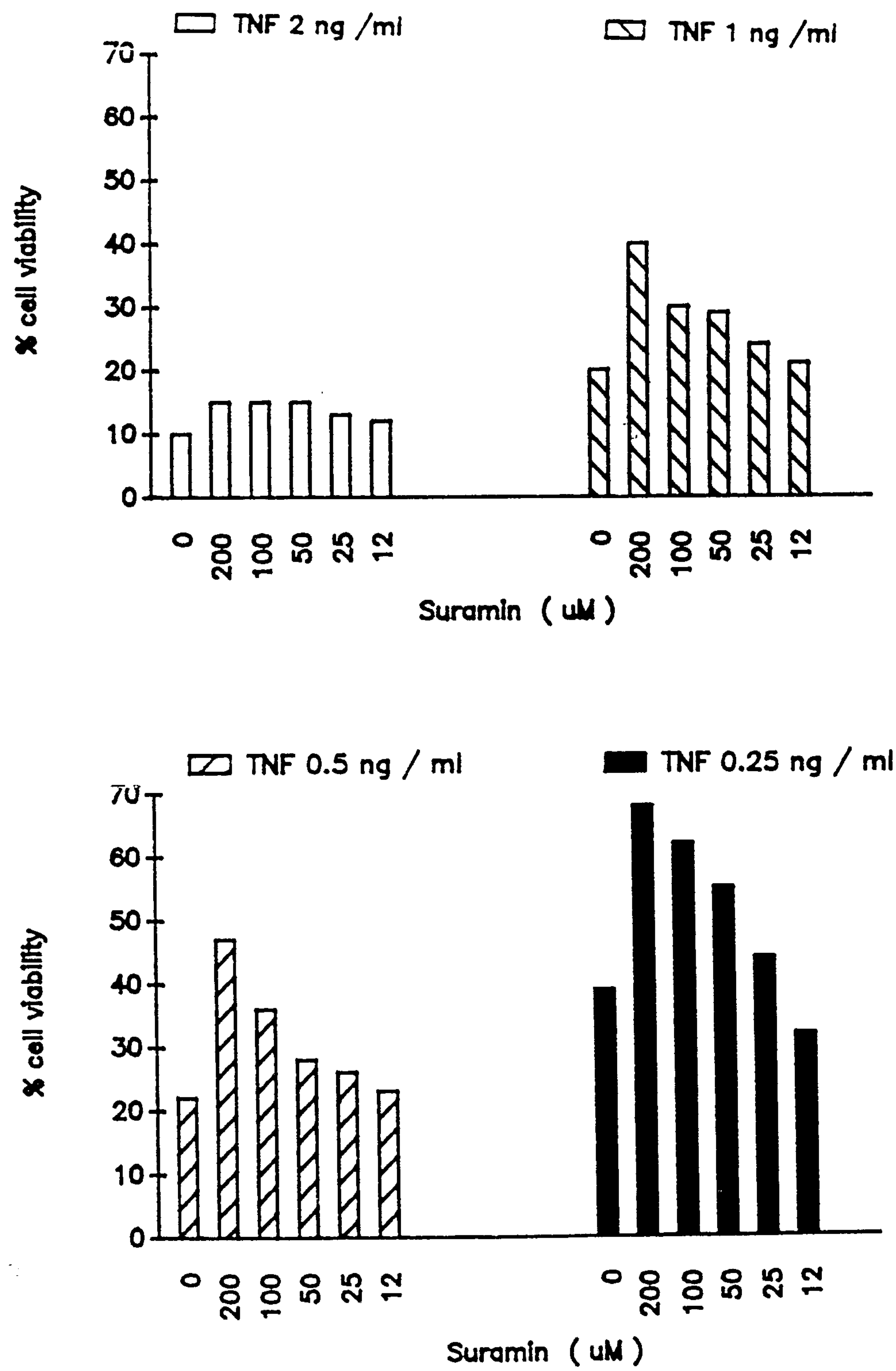


Fig. 5

Effect of Suramin on TNF cytotoxicity on LM cells – ActD

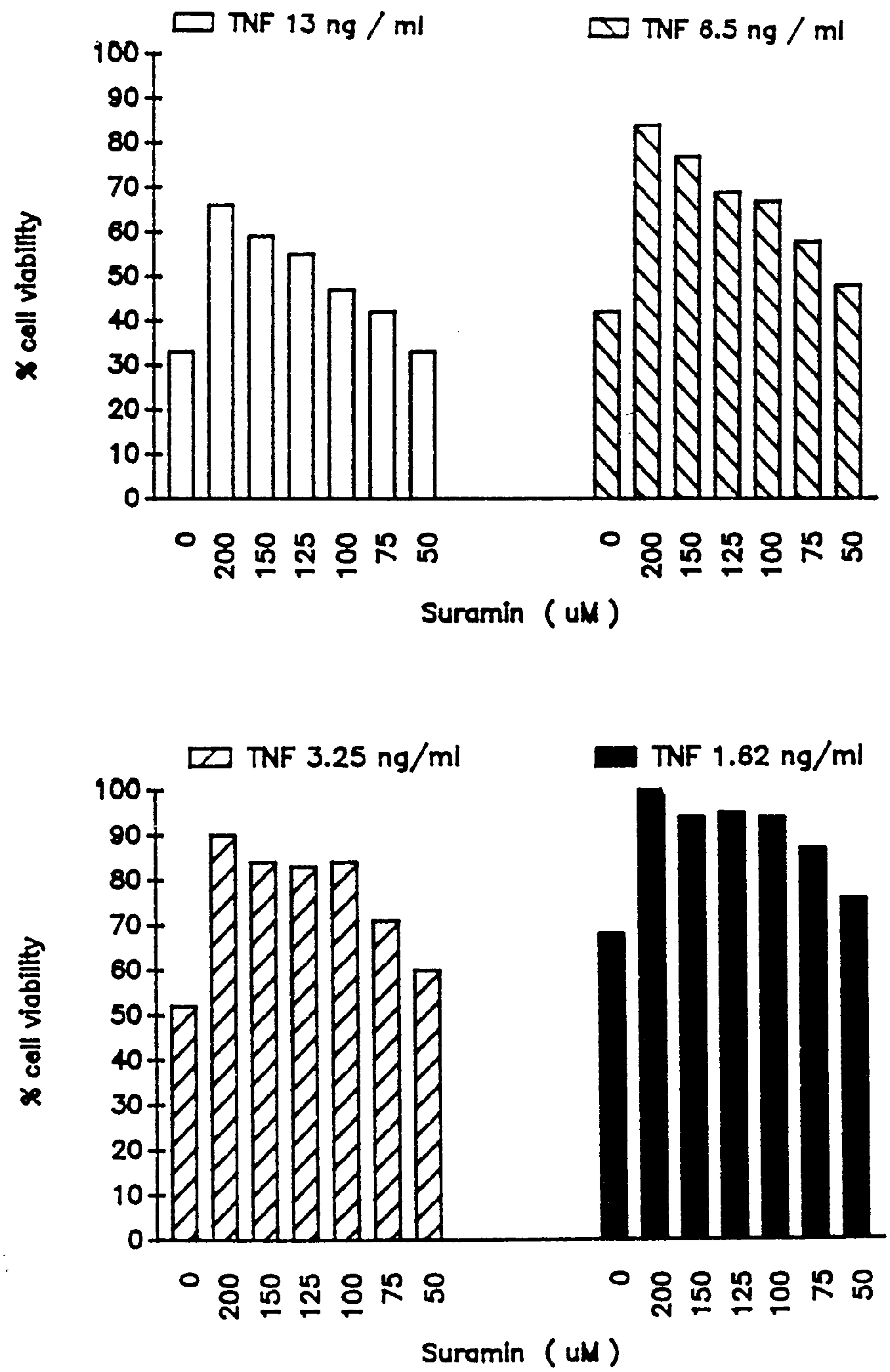


Fig. 6